# EUROPEAN PATENT APPLICATION

(11) **EP 4 574 148 A1**
(43) Date of publication of application: **25.06.2025**
(21) Application number: 23218917.5
(22) Date of filing: 20.12.2023
(51) Int. Cl.: A61K 31/404, A61K 31/4184, A61K 31/4422, A61K 9/00, A61P 9/12, A61P 9/10

(54) **FIXED COMBINATION OF TELMISARTAN, AMLODIPINE AND INDAPAMIDE FOR THE TREATMENT OF HYPERTENSION**

(71) Applicant: KRKA, d.d., Novo mesto, 8000 Novo mesto (SI)
(72) Inventor: Kos, Jernej, 8000 Novo mesto (SI); Rekic, Aleksander, 8000 Novo mesto (SI)
(74) Representative: Ter Meer Steinmeister & Partner

(57) **Abstract**

The present invention relates to a fixed combination medicinal product that is highly efficient and safe when being used in treating essential hypertension or/and reducing the risk of development of a condition associated with essential hypertension, and that additionally ensures excellent patient compliance. The fixed combination medicinal product comprises (a) telmisartan at a dose of 40 mg, amlodipine at a dose of 5 mg, and indapamide at a dose of 1.5 mg for modified release or at a dose of 2.5 mg for immediate release, (b) telmisartan at a dose of 80 mg, amlodipine at a dose of 5 mg, and indapamide at a dose of 1.5 mg for modified release or at a dose of 2.5 mg for immediate release, or (c) telmisartan at a dose of 80 mg, amlodipine at a dose of 10 mg, and indapamide at a dose of 1.5 mg for modified release or at a dose of 2.5 mg for immediate release. The fixed combination medicinal product is administered once or twice, preferably once a day and is used as (α) a first-line antihypertensive therapy for a hypertensive patient, (β) a second-line antihypertensive therapy for a hypertensive patient who is a non-responder to a previous antihypertensive therapy, or (γ) a substitution antihypertensive therapy for a hypertensive patient whose blood pressure is adequately controlled by a previous antihypertensive therapy.

## Description

### Field of the invention

The present invention relates to a fixed combination medicinal product for use in treating hypertension or for reducing the risk of development of a condition associated with hypertension in a hypertensive subject. In particular, the present invention relates to a fixed combination medicinal product comprising telmisartan, amlodipine and indapamide. In particular, the present invention relates to a fixed combination medicinal product comprising telmisartan, amlodipine and indapamide preferably in an amount of 40-80 mg of telmisartan, 5-10 mg of amlodipine and 1.5 - 2.5 mg of indapamide.

### Background art

Hypertension, also commonly known as high blood pressure, is a medical condition in which the arterial blood pressure is persistently elevated and which represents a major cause of premature death worldwide. According to an estimation provided on the website of the World Health Organization (WHO) in early 2023, about 1.28 billion adults aged 30-79 years worldwide suffer from hypertension, with two-thirds of them living in low- and middleincome countries and only about 46% being aware of their medical condition.

Risk factors for the development of hypertension include both modifiable risk factors such as physical inactivity, unhealthy diets, consumption of tobacco and alcohol, and nonmodifiable risk factors such as a family history of hypertension and co-existing diseases such as diabetes or kidney disease. Despite the existence of effective and affordable techniques, hypertension is not always well detected, especially since it is usually not accompanied by any symptoms. Only in case of very high blood pressures can symptoms such as severe headaches, chest pain, dizziness, nausea, blurred vision, anxiety and abnormal heart rhythm be experienced.

Although the manipulation of modifiable risk factors can help to lower the blood pressure, most subjects suffering from hypertension require antihypertensive drug treatment alongside lifestyle modifications. In the 2023 hypertension guidelines of the European Society of Hypertension (ESH), five major drug classes are recommended as first-line agents for antihypertensive drug treatment, namely angiotensin-converting-enzyme inhibitors, angiotensin receptor blockers, calcium channel blockers, thiazide/thiazide-like diuretics and beta blockers. These drugs can be used alone or in combination.

Typically, therapy is initiated with monotherapy or with a two-drug combination and switched to a three-drug combination if, upon administration of the maximum recommended and tolerated dose of both drugs, the blood pressure of the hypertensive subject cannot be adequately controlled with the initial two-drug combination. Preferred two-drug combinations comprise a renin-angiotensin system (RAS) blocker combined with a calcium channel blocker (CCB) or a thiazide/thiazide-like diuretic, even though other combinations of the five major drug classes can also be used (Mancia et al., "2023 ESH Guidelines for the management of arterial hypertension", J. Hypertension 41(12) (2023), 1874-2071).

If the use of a three-drug combination becomes necessary, it is recommended that the combination comprises each of a RAS blocker, a calcium channel blocker and a thiazide/thiazide-like diuretic, wherein the RAS blocker may be either an angiotensin-converting-enzyme (ACE) inhibitor or an angiotensin receptor blocker (ARB). The rationale behind this is that such three-drug combinations typically result in an antihypertensive effect that is considerably higher than that of the individual components (Volpe et al., "Rationale for triple fixed-dose combination therapy with an angiotensin II receptor blocker, a calcium channel blocker, and a thiazide diuretic", Vase. Health Risk Manag. 8 (2012), 371-380), resulting in adequate control of the blood pressure in up to 90% of the patients (Mancia et al., "2023 ESH Guidelines for the management of arterial hypertension", J. Hypertension 41(12) (2023), 1874-2071).

Specific examples of suitable angiotensin receptor blockers comprise azilsartan, candesartan, eprosartan, irbesartan, losartan, olmesartan, telmisartan and valsartan. As specific examples of calcium channel blockers, benzothiazepines such as diltiazem, dihydropyridines such as amlodipine, felodipine, nifedipine and nitrendipine, and phenylalkylamines such as verapamil are adopted. Thiazide/thiazide-like diuretics suitable for use in a three-drug combination comprise, among others, chlortalidone, hydrochlorothiazide, indapamide and xipamide.

Considering the plurality of possible combinations conferred by the aforementioned lists of compounds, the provision of an efficient and simultaneously safe antihypertensive therapy employing each of an angiotensin receptor blocker, a calcium channel blocker and a thiazide/thiazide-like diuretic is still a demanding task. De la Sierra et al. ("Blood pressure control with angiotensin receptor blocker-based three-drug combinations: key trials", Adv. Ther. 29(5) (2012), 401-415) review two clinical studies in which patients with moderate to severe hypertension were administered with three-drug combinations employing either olmesartan or valsartan as the angiotensin receptor blocker, amlodipine as the calcium channel blocker, and hydrochlorothiazide as the thiazide/thiazide-like diuretic.

On the other hand, WO 2018/138578 A1 suggests a clinical study in which subjects with Grade 1 and 2 essential hypertension are administered a pharmaceutical composition comprising telmisartan as the angiotensin receptor blocker, amlodipine besylate as the calcium channel blocker, and indapamide as the thiazide/thiazide-like diuretic. Since single-pill combination (SPC) therapy is known to lead to improved adherence and persistence compared with free-equivalent combination (FEC) therapy and thereby achieves better blood pressure control (Parati et al., "Adherence to single-pill versus free-equivalent combination therapy in hypertension", Hypertension 77 (2021), 692-705), the three-drug combination of WO 2018/138578 A1 is provided in the form of a single pill.

### Summary of the invention

### Problem to be solved

In the clinical study of WO 2018/138578 A1, either a three-drug combination pill comprising 10 mg of telmisartan, 1.25 mg of amlodipine besylate and 0.625 mg of indapamide (corresponding to 50% of the lowest hypertension therapeutic dose of each drug, LHTD), or a three-drug combination pill comprising 20 mg of telmisartan, 2.5 mg of amlodipine besylate and 1.25 mg of indapamide (corresponding to 100% of the LHTD of each drug) is tested.

By using such low-dose combinations of telmisartan, amlodipine and indapamide, the authors try to combine the individual therapeutic effects of telmisartan, amlodipine and indapamide and to thereby provide an efficacious treatment of hypertension that can be used as a first-line antihypertensive therapy while simultaneously avoiding or ameliorating unwanted side effects associated with high dosing. In particular, peripheral edema is a frequent and dose-dependent adverse event (AE) of amlodipine therapy. By reducing the drug amount to 50% of the LHTD, the AE profile was improved by the inventors of WO 2018/138578 A1.

However, there is still a need for improving the patient's clinical benefit. In particular, it would be desirable to further improve efficacy of an antihypertensive therapy using an antihypertensive drug combination while simultaneously maintaining an acceptable level of adverse events/side effects. Furthermore, it would be desirable to provide an antihypertensive therapy additionally ensuring good patient compliance.

### Means for solving the problem

In order to solve the aforementioned problem, the present inventors carried out intense studies and unexpectedly found that there is no need for substituting drug components or for adding additional drug components to the 50% LHTD three-drug combination of WO 2018/138578 A1. Instead, by increasing the dosage in fixed combination of telmisartan, amlodipine and indapamide to a range of 200 to 400% LHTD, it is possible to further improve the efficacy of an antihypertensive therapy while not substantially affecting safety issues.

In particular, it has been surprisingly found that, by administering each of telmisartan, amlodipine and indapamide at a dose as mentioned above, it is possible to provide a highly efficacious treatment having an advantageous low side effect profile, e.g. as mentioned in WO 2018/138578 A1 for 50% of the LHTD. In particular, adverse events such as acute cardiac decompensation, cellular lysis (e.g. acute limb ischemia, rhabdomyolysis, extend trauma), dehydration, especially edema, metabolic acidosis, worsening of renal function, and sudden worsening of renal condition (e.g. infectious diseases) are occurring advantageously rarely or/and commonly in mild form.

Furthermore, the triple telmisartan/amlodipine/indapamide fixed combination medicinal product, e.g. the monolayer tablet, can provide advantageous safety profile with reduced frequency or severeness of some adverse events, e.g. better safety profile when compared to the telmisartan/amlodipine, telmisartan/indapamide and amlodipine/indapamide dual therapies. The advantageous safety profile may concern the following adverse events abdominal pain, altered bowel habits (including diarrohea and constipation), ankle swelling, asthenia, depression, dyspepsia, dyspnoea, fatigue, flushing, headache, hyperkalaemia, hypokalaemia, hypersensitivity reactions, muscle cramps, nausea, oedema, palpitations, rash, somnolence, visual disturbance. Other possible adverse events include: abnormal hepatic function, acute angle-closure glaucoma, agranulocytosis, allergic reactions, alopecia, anaemia, anaphylactic reaction, angioedema, anxiety, aplastic anaemia, arrhythmia (including bradycardia, ventricular tachycardia and atrial fibrillation), arthralgia, back pain, blood creatine phosphokinase increased, blood creatinine increased, blood uric acid increased, blurred vision, chest pain, choroidal effusion, confusion, cough, dizziness, drug eruption, dry mouth, dysgeusia, eczema, electrocardiogram QT prolonged, elevated liver enzyme levels, eosinophilia, erythema, erythema multiforme, exanthema, exfoliative dermatitis, extrapyramidal disorder, flatulence, gastritis, gingival hyperplasia, gynecomastia, haemoglobin decreased, haemolytic anaemia, hepatic enzymes increased, hepatitis, hyperglycaemia, hyperhidrosis, hypertonia, hypochloraemia, hypoesthesia, hypoglycaemia, hypomagnesaemia, hyponatraemia, hypotension, impotence, increased urinary frequency, influenza-like illness, insomnia, interstitial lung disease, jaundice, leukocytopenia, malaise, micturition disorder, mood changes (including anxiety), muscular weakness, myalgia, myocardial infarction, myopia, nocturia, orthostatic hypotension, pain, pain in extremity, pancreatitis, paresthesia, peripheral neuropathy, photosensitivity, possibility of onset of hepatic encephalopathy in case of hepatic insufficiency, possible worsening of pre-existing acute disseminated lupus erythematosus, pruritus, purpura, Quincke oedema, renal failure, renal impairment including acute renal failure, rhabdomyolysis, rhinitis, sepsis including fatal outcome, skin discolouration, Stevens-Johnson syndrome, stomach discomfort, syncope, tendon pain (tendinitis like symptoms), thrombocytopenia, tinnitus, torsade de pointes, toxic epidermal necrolysis, toxic skin eruption, tremor, upper respiratory tract infection including pharyngitis and sinusitis, urinary tract infection including cystitis, urticaria, vasculitis, vertigo, vomiting, weight decrease and weight increase.

Hence, the clinical benefit for the patient was unexpectedly improved. Surprisingly, the clinical benefit was particularly advantageous for patients suffering from Grade 1 or 2 hypertension, and especially Grade 2 hypertension.

Furthermore, it has unexpectedly turned out that a product comprising each of telmisartan, amlodipine and indapamide at a dose as mentioned above does not only efficiently and safely reduce the blood pressure of a hypertensive patient but additionally lowers blood glucose and/or glycated haemoglobin. Therefore, a three-drug combination of telmisartan, amlodipine and indapamide can also be used for treating diabetes or/and for reducing the risk of development of diabetes and can be conveniently administered to a hypertensive patient diagnosed with prediabetes or diabetes.

Finally, it has been recognized by the inventors that, by providing a three-drug combination of telmisartan, amlodipine and indapamide in the form of a monolayer tablet having the three antihypertensive agents combined in a single layer, it is additionally possible to reduce treatment complexity, and to thereby reliably increase patient compliance and blood pressure control. By this means, the three-drug combination is highly suitable for antihypertensive therapy, and in particular for substitution therapy. The present invention is defined by the claims.

In detail, the present invention thus relates to a fixed combination medicinal product for treating essential hypertension or/and reducing the risk of development of a condition associated with essential hypertension as defined in claim 1. The fixed combination medicinal product comprises:
(a) telmisartan at a dose of 40 mg, amlodipine at a dose of 5 mg, and indapamide at a dose of 1.5 mg for modified release or at a dose of 2.5 mg for immediate release; or
(b) telmisartan at a dose of 80 mg, amlodipine at a dose of 5 mg, and indapamide at a dose of 1.5 mg for modified release or at a dose of 2.5 mg for immediate release; or
(c) telmisartan at a dose of 80 mg, amlodipine at a dose of 10 mg, and indapamide at a dose of 1.5 mg for modified release or at a dose of 2.5 mg for immediate release.

The fixed combination medicinal product is administered once or twice, preferably once a day (to a hypertensive patient in need thereof), and is used as:
(α) a first-line antihypertensive therapy for a hypertensive patient; or
(β) a second-line antihypertensive therapy for a hypertensive patient who is a non-responder to a previous antihypertensive therapy; or
(γ) a substitution antihypertensive therapy for a hypertensive patient whose blood pressure is adequately controlled by a previous antihypertensive therapy.

Preferred embodiments of the fixed combination medicinal product for use of the present invention are described in the subclaims.

### Detailed description of the invention

The invention broadly relates to the use of a fixed combination medicinal product in a method of treating essential hypertension or reducing the risk of development of a condition associated with essential hypertension in a hypertensive subject. The fixed combination medicinal product comprises a total of three active pharmaceutical ingredients (APIs) representing three different drug classes of antihypertensive agents, namely an angiotensin receptor blocker, a calcium channel blocker and a thiazide/thiazide-like diuretic.

Generally, "fixed combination" or "fixed combination medicinal product" denotes two or more active substances within a single pharmaceutical form.

More specifically, the invention makes use of a fixed combination medicinal product comprising telmisartan, amlodipine and indapamide, wherein each of the telmisartan, the amlodipine and the indapamide may be present in the form of the API as such (i.e. in the form of the free API), in the form of a pharmaceutically acceptable salt of the API, in the form of a pharmaceutically acceptable solvate (especially a hydrate) of the API, or in the form of a pharmaceutically acceptable salt solvate (especially a salt hydrate) of the API. According to the invention, each of the three APIs may be either crystalline or amorphous, the term "crystalline" being intended to encompass all possible polymorphs of a given API. In this regard "free API" means that the API is not in salt form, i.e. not protonated or deprotonated.

That is, the term "telmisartan" as used herein designates a compound selected from free telmisartan, a pharmaceutically acceptable salt of telmisartan, a pharmaceutically acceptable solvate of telmisartan, and a pharmaceutically acceptable salt solvate of telmisartan, which compound is preferably amorphous. Free telmisartan (IUPAC name: 2-(4-{[4-methyl-6-(1-methyl-1H-1,3-benzodiazol-2-yl)-2-propyl-1H-1,3-benzodiazol-1-yl]methyl} phenyl)benzoic acid) decreases the systemic vascular resistance by selectively binding to the angiotensin II receptor type 1 (AT₁) and inhibiting binding of the vasoconstrictor angiotensin II thereto.

The term "amlodipine" as used herein designates a compound selected from free amlodipine, a pharmaceutically acceptable salt of amlodipine, a pharmaceutically acceptable solvate of amlodipine, and a pharmaceutically acceptable salt solvate of amlodipine, which compound is preferably crystalline. Free amlodipine (IUPAC name: (RS)-3-ethyl 5-methyl 2-[(2-aminoethoxy)methyl]-4-(2-chlorophenyl)-6-methyl-1,4-dihydropyridine-3,5-dicarboxylate) inhibits calcium ion influx across smooth muscle cell membranes, resulting in vasodilation and a reduction in peripheral vascular resistance.

The term "indapamide" as used herein designates a compound selected from free indapamide, a pharmaceutically acceptable salt of indapamide, a pharmaceutically acceptable solvate of indapamide, and a pharmaceutically acceptable salt solvate of indapamide, which compound is preferably crystalline. Free indapamide (IUPAC name: 4-chloro-N-(2-methyl-2,3-dihydroindol-1-yl)-3-sulfamoylbenzamide) inhibits the sodium-chloride symporter at the proximal segment of the distal convoluted tubule and prevents the kidney from reabsorbing sodium and chloride ions, and finally lowers the blood pressure by increasing urinary excretion.

The terms "pharmaceutically acceptable salt", "pharmaceutically acceptable solvate" and "pharmaceutically acceptable salt solvate" as used herein in connection with the hypertensive agents of the fixed combination medicinal product to be used in the invention designate a salt, a solvate or a salt solvate that has been approved or is approvable by a regulatory authority such as the European Medicines Agency (EMA) or the U.S. Food and Drug Administration (FDA), or/and that is listed in a generally recognized pharmacopoeia such as the European Pharmacopoeia or the U.S. Pharmacopoeia and has been found to be acceptable for medical use in humans or animals.

Specific examples of pharmaceutically acceptable salts are mentioned in "Remington: The Science and Practice of Pharmacy" (20th Edition, edited by A.R. Gennaro, Lippincott Williams & Wilkins, Baltimore and Philadelphia, 2000) and comprise, among others, acetate, besylate, bromide, chloride, citrate, fumarate, glucuronate, hydrobromide, hydrochloride, maleate, mesylate, nitrate, phosphate, potassium, sodium, succinate, sulfate, tartrate and tosylate. Specific examples of pharmaceutically acceptable solvates and pharmaceutically acceptable salt solvates are known to a person skilled in the art and particularly include hydrates, which hydrates may contain either stoichiometric or non-stoichiometric amounts of water.

According to the invention, the fixed combination medicinal product can be used at different dosages of the active pharmaceutical ingredients (APIs).

In embodiment (a), the fixed combination medicinal product to be used in the invention comprises telmisartan at a dose of 40 mg (in terms of free telmisartan), amlodipine at a dose of 5 mg (in terms of free amlodipine), and indapamide at a dose of 1.5 mg for modified release or at a dose of 2.5 mg for immediate release (in terms of free indapamide). In this embodiment, it is preferred that indapamide is formulated in such a manner that, upon administration to a patient, it is immediately released. Accordingly, the fixed combination medicinal product of embodiment (a) thus preferably comprises telmisartan at a dose of 40 mg (in terms of free telmisartan), amlodipine at a dose of 5 mg (in terms of free amlodipine), and indapamide at a dose of 2.5 mg (in terms of free indapamide).

In embodiment (b), the fixed combination medicinal product comprises telmisartan at a dose of 80 mg (in terms of free telmisartan), amlodipine at a dose of 5 mg (in terms of free amlodipine), and indapamide at a dose of 1.5 mg for modified release or at a dose of 2.5 mg for immediate release (in terms of free indapamide). In this embodiment, it is preferred that indapamide is formulated in such a manner that, upon administration to a patient, it is immediately released. Accordingly, the fixed combination medicinal product of embodiment (b) preferably comprises telmisartan at a dose of 80 mg (in terms of free telmisartan), amlodipine at a dose of 5 mg (in terms of free amlodipine), and indapamide at a dose of 2.5 mg (in terms of free indapamide).

In embodiment (c), the fixed combination medicinal product comprises telmisartan at a dose of 80 mg (in terms of free telmisartan), amlodipine at a dose of 10 mg (in terms of free amlodipine), and indapamide at a dose of 1.5 mg for modified release or at a dose of 2.5 mg for immediate release (in terms of free indapamide). In this embodiment, it is preferred that indapamide is formulated in such a manner that, upon administration to a patient, it is immediately released. Accordingly, the fixed combination medicinal product of embodiment (c) preferably comprises telmisartan at a dose of 80 mg (in terms of free telmisartan), amlodipine at a dose of 10 mg (in terms of free amlodipine), and indapamide at a dose of 2.5 mg (in terms of free indapamide).

The term "immediate release" (IR) as used herein designates a release profile of an API which, upon subjecting the fixed combination medicinal product for 30 minutes to an *in vitro* dissolution test in accordance with European Pharmacopoeia (paddle apparatus, 100 rpm, 900 ml phosphate buffer pH 6.8, 37°C), correlates with a release of at least 70% by weight, preferably at least 80% by weight, and more preferably at least 90% by weight of the API, based on the total amount of the API contained in the fixed combination medicinal product. According to the invention, it is particularly preferred that each of telmisartan, amlodipine and indapamide is formulated for immediate release.

In contrast thereto, the term "modified release" (MR) as used herein designates a release profile of an API which, upon subjecting the fixed combination medicinal product for 60 minutes the aforementioned dissolution test, correlates with a release of not more than 50% by weight, preferably not more than 40% by weight, and more preferably not more than 30% by weight of the API, based on the total amount of the API contained in the fixed combination medicinal product. The term "modified release" as used herein is meant to comprise delayedrelease (DR), extended-release (ER) and sustained release (SR) formulations.

In a preferred embodiment, the fixed combination medicinal product comprises:
(b') telmisartan at a dose of 80 mg (in terms of free telmisartan), amlodipine at a dose of 5 mg (in terms of free amlodipine), and indapamide at a dose of 2.5 mg for immediate release (in terms of free indapamide); or
(c') telmisartan at a dose of 80 mg (in terms of free telmisartan), amlodipine at a dose of 10 mg (in terms of free amlodipine), and indapamide at a dose of 2.5 mg for immediate release (in terms of free indapamide).

For avoidance of doubt it should be noted that within that application "in terms of free API" means, that the respective amount refers to the amount of free API. If the API is administered e.g. in salt form, the weight of the counterion has to be added. For example, if amlodipine is administered in form of the besylate salt, a dose of 10 mg (in terms of free amlodipine) corresponds to 13.87 mg amlodipine besylate.

According to the invention, the fixed combination medicinal product referred to above is administered to a hypertensive patient.

Based on the definition given in the 2023 hypertension guidelines of the ESH (Mancia et al., "2023 ESH Guidelines for the management of arterial hypertension", J. Hypertension 41(12) (2023), 1874-2071), the term "hypertensive patient" as used herein designates a human patient who repeatedly experiences a sitting systolic blood pressure (SBP) value of ≥ 140 mmHg or/and a sitting diastolic blood pressure (DBP) value of ≥ 90 mmHg.

In this context, it is intended that SBP values of 140-159 mmHg or/and DBP values of 90-99 mmHg are indicative of Grade 1 hypertension, SBP values of 160-179 mmHg or/and DBP values of 100-109 mmHg are indicative of Grade 2 hypertension, and SBP values of ≥ 180 mmHg or/and DBP values of ≥ 110 mmHg are indicative of Grade 3 hypertension. The human patient may be a pediatric patient (age < 18 years) or an adult patient (age ≥ 18 years), the term "adult patient" also including a geriatric patient (age ≥ 65 years).

In a preferred embodiment, however, the hypertensive patient to be treated with the fixed combination medicinal product is an adult patient, and in particular and adult patient having, without pharmacological interventions, a sitting SBP value of from 140 to 180 mmHg or/and a sitting DBP value from 90 to 110 mmHg. More preferably, the adult patient has a sitting SBP value of from 160 to 179 mmHg or/and a sitting DBP value of from 100 to 109 mmHg.

In accordance with the definitions given in the 2023 hypertension guidelines of the ESH (Mancia et al., "2023 ESH Guidelines for the management of arterial hypertension", J. Hypertension 41(12) (2023), 1874-2071), it is thus preferred that the fixed combination medicinal product described herein is administered to an adult patient diagnosed with Grade 1 or Grade 2 hypertension, even though the treatment of adult patients diagnosed with Grade 3 hypertension is envisioned by the invention as well.

The fixed combination medicinal product is administered once a day or twice a day. In a preferred embodiment the administration regimen is once a day. By administering the fixed combination medicinal product to a hypertensive patient once or twice a day and for a specified period of time, it is possible to treat essential hypertension, the term "essential hypertension" being known in the art and meaning that the hypertension has no obvious cause. The terms "treat" and "treatment" as used herein designate an eradication or an amelioration of essential hypertension (coinciding with a reduction of the SBP value or/and the DBP value relative to the respective value before the start of treatment with the fixed combination medicinal product), or of one or more symptoms associated with essential hypertension.

Since hypertension is a condition in which the blood pressure in the arterial system is persistently and chronically elevated, the fixed combination medicinal product is typically used for long-term treatment and is administered over a prolonged period of time. The administration period can be suitably chosen by a physician in accordance with needs, especially based on the grade of hypertension experienced by a hypertensive patient.

In general, the administration period is in the range of from a few weeks, e.g. 4 weeks or 8 weeks, to more than 1 year. Typically, however, the fixed combination medicinal product is administered as long as it is well tolerated and achieves adequate control of the blood pressure, the term "adequate control" as used herein meaning that the blood pressure of the hypertensive patient is controlled at a sitting SBP value of < 140 mmHg and at a sitting DBP value of < 90 mmHg.

According to the invention, the fixed combination medicinal product can be used at different stages of antihypertensive therapy or/and can address different patient groups, i.e. can be administered to naive or pre-treated hypertensive patients.

In embodiment (α), the fixed combination medicinal product is used as a first-line antihypertensive therapy. This may mean a therapy for a hypertensive patient with newly diagnosed essential hypertension, i.e. a hypertensive patient who has not received any antihypertensive therapy before (also called naive hypertensive patient).

In embodiment (β), the fixed combination medicinal product is used as a second-line antihypertensive therapy for a hypertensive patient who is a non-responder to a previous antihypertensive therapy, i.e. a hypertensive patient who has received a previous antihypertensive therapy but has not responded thereto. In this context, the term "non-responder" as used herein designates a patient whose sitting SBP value or/and sitting DBP value does not decrease by at least 5% after having received one or more antihypertensive agents once or twice, preferably once a day over a period of 6 weeks.

In this embodiment, the previous antihypertensive therapy is typically a first-line antihypertensive therapy and may include administration of one or more antihypertensive agents, and in particular two antihypertensive agents. Preferably, the previous antihypertensive therapy is a first-line antihypertensive therapy and includes administration of two or more antihypertensive agents, and in particular administration of two antihypertensive agents.

In embodiment (γ), the fixed combination medicinal product is used as a substitution antihypertensive therapy for a hypertensive patient whose blood pressure is adequately controlled by a previous antihypertensive therapy, i.e. a hypertensive patient who has received a previous antihypertensive therapy and has suitably responded thereto by having its blood pressure lowered to a sitting SBP value of < 140 mmHg and at a sitting DBP value of < 90 mmHg.

In this embodiment, the previous antihypertensive therapy may be a first-line antihypertensive therapy or a second-line antihypertensive therapy and may include administration of one or more antihypertensive agents, and in particular two or more antihypertensive agents. Preferably, the previous antihypertensive therapy is a second-line antihypertensive therapy and includes administration of two or more antihypertensive agents, and in particular administration of three antihypertensive agents.

The above-described embodiments can be combined. For example, the dosage (a) can be combined with scheme/patient group (α). Further combinations are (a)+(β), (a)+(γ), (b)+(α), (b)+(β), (b)+(γ), (c)+(α), (c)+(β) and (c)+ (γ). In a preferred embodiment, the invention encompasses combination (a)+(γ) but does not encompass the combinations (a)+(α) and (a)+(β). Preferred combinations are (b')+(α), (b')+(β), (b')+(γ), (c')+(α), (c')+(β) and (c')+(γ). Further, as outlined below in more detail, scheme/patient group (γ) is a preferred one.

In the preferred embodiment (γ), the fixed combination medicinal product is used as a substitution antihypertensive therapy for a hypertensive patient whose blood pressure is adequately controlled by a previous antihypertensive therapy. While the invention basically provides for the possibility of the previous antihypertensive therapy being a first-line antihypertensive therapy, it is particularly preferred that the previous antihypertensive therapy is a second-line antihypertensive therapy.

In a preferred embodiment, the fixed combination medicinal product is used as a substitution antihypertensive therapy for a hypertensive patient whose blood pressure is adequately controlled by a previous second-line antihypertensive therapy, wherein the fixed combination medicinal product administered once a day or twice a day, preferably once a day, comprises the fixed combination medicinal product defined in the aforementioned embodiment (a).

In a preferred embodiment, the fixed combination medicinal product is used as a substitution antihypertensive therapy for a hypertensive patient whose blood pressure is adequately controlled by a previous second-line antihypertensive therapy, wherein the fixed combination medicinal product administered once or twice, preferably once a day comprises the fixed combination medicinal product defined in the aforementioned embodiment (b).

In a preferred embodiment, the fixed combination medicinal product is used as a substitution antihypertensive therapy for a hypertensive patient whose blood pressure is adequately controlled by a previous second-line antihypertensive therapy, wherein the fixed combination medicinal product administered once or twice, preferably once a day comprises the fixed combination medicinal product defined in the aforementioned embodiment (c).

In a preferred embodiment, the previous antihypertensive therapy comprises administration of two or more antihypertensive agents, wherein the use of angiotensin receptor blockers, calcium channel blockers, thiazide/thiazide-like diuretics and combinations thereof is preferred. More preferably, the previous antihypertensive therapy comprises administration of at least one antihypertensive agent selected from telmisartan, amlodipine and indapamide.

In a preferred embodiment, the previous antihypertensive therapy comprises administration of at least two antihypertensive agents selected from telmisartan, amlodipine and indapamide, i.e. a fixed or non-fixed combination of telmisartan and amlodipine, a fixed or non-fixed combination of telmisartan and indapamide, a fixed or non-fixed combination of amlodipine and indapamide, or a fixed or non-fixed combination of telmisartan, amlodipine and indapamide.

In a preferred embodiment, the previous antihypertensive therapy comprises administration of two antihypertensive agents selected from telmisartan, amlodipine and indapamide, i.e. a fixed or non-fixed combination of telmisartan and amlodipine, a fixed or non-fixed combination of telmisartan and indapamide, and a fixed or non-fixed combination of amlodipine and indapamide. The previous antihypertensive therapy is preferably a second-line antihypertensive therapy.

In a preferred embodiment, the previous antihypertensive therapy comprises administration of a fixed or non-fixed combination of telmisartan, amlodipine and indapamide. More preferably, the previous antihypertensive therapy comprises administration of a non-fixed combination of telmisartan, amlodipine and indapamide, wherein the previous antihypertensive therapy is preferably a second-line antihypertensive therapy.

In a preferred embodiment the fixed combination medicinal product of the present invention is for use in treating essential hypertension or/and reducing the risk of development of a condition associated with essential hypertension,
wherein the treated patient population shows significant increase in proportion of patients with sitting diastolic blood pressure (SiDBP) lower than 90 mmHg and/or SiDBP reduction of ≥ 10 mmHg , e.g. over 8 and 16 weeks, after taking the treatment of the invention compared to a therapy with a dual drug combination, e.g. telmisartan and amlodipine;
wherein the treated patient population shows significant increase in proportion of patients with sitting systolic blood pressure SiSBP lower than 140 mmHg and/or SiSBP reduction of ≥ 20 mmHg, e.g. over 8 and 16 weeks, after taking the treatment of the invention compared to a therapy with a dual drug combination, e.g. telmisartan and amlodipine; and/or
wherein the treated patient population shows significant increase in proportion of patients with Sitting blood pressure normalization (SiSBP / SiDBP lower than 140/90 mmHg), e.g. over 8 and 16 weeks, after taking the treatment of the invention compared to a therapy with a dual drug combination, e.g. telmisartan and amlodipine.

In an embodiment the combination product of the present invention can be used for patients which are regarded as difficult to treat. In an embodiment thus the hypertensive patient is treated despite having a history of at least one of the following conditions:
- hypertensive encephalopathy, stroke or transient ischemic attack;
- acute coronary syndrome, coronary artery disease, coronary artery bypass graft, myocardial infarction, percutaneous transluminal coronary revascularization or unstable angina;
- persistent or permanent atrial fibrillation;
- congestive heart failure;
- primary aldosteronism, renal artery stenosis, pheochromocytoma or Cushing's syndrome;
- anuria, end-stage renal disease, or estimated glomerular filtration rate (eGFR) of ≤ 60 ml/min/1.73m²;
- serum creatinine level of ≥ 1.5 times the upper limit of normal range;
- aspartate aminotransferase level or alanine aminotransferase level ≥ 3 times the upper limit of normal range; and
- alcohol or drug abuse.

In an embodiment, the hypertensive patient to be treated has a history of hypertensive encephalopathy, stroke or transient ischemic attack.

In an embodiment, the hypertensive patient has a history of acute coronary syndrome, coronary artery disease, coronary artery bypass graft, myocardial infarction, percutaneous transluminal coronary revascularization or unstable angina.

In an embodiment, the hypertensive patient has a history of persistent or permanent atrial fibrillation.

In an embodiment, the hypertensive patient has a history of congestive heart failure.

In an embodiment, the hypertensive patient has a history of primary aldosteronism, renal artery stenosis, pheochromocytoma or Cushing's syndrome.

In an embodiment, the hypertensive patient has a history of anuria, end-stage renal disease, or estimated glomerular filtration rate (eGFR) of ≤ 60 ml/min/1.73m².

In an embodiment, the hypertensive patient has a history of serum creatinine level of ≥ 1.5 times the upper limit of normal range.

In an embodiment, the hypertensive patient has a history of aspartate aminotransferase level or alanine aminotransferase level ≥ 3 times the upper limit of normal range.

In an embodiment, the hypertensive patient has a history of alcohol or drug abuse.

As mentioned above it was unexpectedly found that the fixed combination of the present invention can be advantageously used for hypertensive patients suffering from a diabetic disorder. Hence, in an embodiment, the hypertensive patient is a prediabetic patient or a diabetic patient, in particular a type 2 diabetic patient. Surprisingly, it has turned out that the fixed combination medicinal product described herein does not only efficiently and safely lower the arterial blood pressure but additionally lowers blood glucose in a patient diagnosed with prediabetes or diabetes.

In particular, it has been found that once daily or twice daily, preferably once daily administration of the fixed combination medicinal product results in a drop in blood glucose level or/and glycated hemoglobin level relative to the respective levels observed before the start of treatment with the fixed combination medicinal product, especially when being compared to a dual-drug combination such as a combination of telmisartan and amlodipine.

By lowering blood glucose, the fixed combination medicinal product reduces the risk of development of diabetes in a non-diabetic or a prediabetic patient and even allows for the treatment of metabolic syndrome and insulin resistance in patients with normal blood pressure (i.e. patients with a sitting SBP value of < 140 mmHg and a sitting DBP value of < 90 mmHg).

In an embodiment, the hypertensive patient is a diabetic patient who has a history of substantially uncontrolled diabetes, in particular a HbA1c level of > 11.0%, within the last three months before the start of treatment with the fixed combination medicinal product.

In an embodiment, administration of the fixed combination medicinal product is interrupted when the hypertensive patient experiences at least one of the following conditions:
- pregnancy or a positive pregnancy test;
- serum sodium level of ≤ 132 mmol/l or ≥ 148 mmol/l;
- serum potassium level of ≤ 3.1 mmol/l or ≥ 5.6 mmol/l; and
- emergency treatment with digoxin, lithium, aliskiren, ritonavir, ketoconazole, diltiazem, simvastatin or an immunosuppressant.

In an embodiment, the hypertensive patient experiences pregnancy or a positive pregnancy test.

In an embodiment, the hypertensive patient experiences serum sodium level of ≤ 132 mmol/l or ≥ 148 mmol/l.

In an embodiment, the hypertensive patient experiences serum potassium level of ≤ 3.1 mmol/l or ≥ 5.6 mmol/l.

In an embodiment, the hypertensive patient experiences emergency treatment with digoxin, lithium, aliskiren, ritonavir, ketoconazole, diltiazem, simvastatin or an immunosuppressant.

Since the aforementioned conditions are deemed to correlate with an increased risk for the development of harmful side effects, administration of the fixed combination medicinal product to a hypertensive patient is preferably interrupted when the patient experiences one or more of the conditions.

Alternatively or additionally to the treatment of essential hypertension as described hereinbefore, the fixed combination medicinal product can be used for reducing the risk of development of a condition associated with essential hypertension, i.e. of a condition that possibly develops when a hypertensive patient is not undergoing any antihypertensive therapy or receives an antihypertensive therapy that does not allow for adequate control of the blood pressure.

In an embodiment, such a condition associated with essential hypertension is a cardiovascular event. Preferably, the condition associated with essential hypertension is a cardiovascular event selected from acute coronary syndrome, coronary artery disease, coronary artery bypass graft, hypertensive encephalopathy, myocardial infarction, percutaneous transluminal coronary revascularization, stroke, transient ischemic attack and unstable angina.

The fixed combination medicinal product may comprise, in addition to telmisartan, amlodipine and indapamide, one or more other active pharmaceutical ingredients that are deemed to provide a clinical benefit to the hypertensive patient and are found to be compatible with the proposed antihypertensive therapy.

By avoiding the presence of drugs that belong to one of the aforementioned classes and that may affect the blood pressure, it is possible to better control administration of the fixed combination medicinal product and to increase efficacy thereof. In addition, by excluding drugs that interact with one or more of telmisartan, amlodipine and indapamide, it is possible to increase safety of the fixed combination medicinal product.

In a preferred embodiment, the fixed combination medicinal product comprises telmisartan, amlodipine and indapamide as the sole active ingredients. This means that the fixed combination medicinal product does not include any active pharmaceutical ingredient other than telmisartan, amlodipine and indapamide.

In a preferred embodiment, the fixed combination medicinal product comprises free telmisartan.

In a preferred embodiment, the fixed combination medicinal product comprises a pharmaceutically acceptable salt of amlodipine, in particular amlodipine besylate.

In a preferred embodiment, the fixed combination medicinal product comprises free indapamide.

In a preferred embodiment, the fixed combination medicinal product comprises free telmisartan, amlodipine besylate and free indapamide.

The fixed combination medicinal product may further comprise, in addition to telmisartan, amlodipine and indapamide, one or more pharmaceutically acceptable excipients. Pharmaceutically acceptable excipients are known to a person skilled in the art and comprise, for instance, binders, diluents, disintegrants fillers, flavours, glidants, lubricants, preservatives, surfactants and sweeteners as disclosed e.g. in US 2006/0045865 A1, "Lexikon der Hilfsstoffe für Pharmazie, Kosmetik und angrenzende Gebiete" (5th Edition, edited by H.P. Fiedler, Editio Cantor Verlag, Aulendorf, 2001) and "Handbook of Pharmaceutical Excipients" (3rd Edition, edited by A.H. Kibbe, Pharmaceutical Press, London, 2000).

The fixed combination medicinal product described herein can be provided in any form that is deemed to be suitable for the administration of telmisartan, amlodipine and indapamide to a patient. In a preferred embodiment, however, the fixed combination medicinal product is formulated for oral delivery so as to simply administer the active pharmaceutical ingredients and to increase patient compliance. Suitable oral dosage forms comprise, but are not limited to, capsules, granules, powders, pastes, solutions, suspensions, syrups, tablets and troches, with capsules and tablets being preferred.

In a preferred embodiment, the fixed combination medicinal product is formulated as a tablet, examples of which comprise chewable tablets, coated tablets, effervescent tablets, lozenges, non-coated tablets and orally disintegrating tablets. The tablet can, for example, be a tablet-in-tablet or a layered tablet such as a monolayer tablet, a bilayer tablet or a triple layer tablet, wherein a monolayer tablet having telmisartan, amlodipine and indapamide combined in a single layer is even more preferred.

In order to achieve a quick therapeutic response, the fixed combination medicinal product can be formulated as immediate release or modified release (e.g. sustained release) dosage form. In a preferred embodiment it is formulated as an immediate release tablet, in particular as a monolayer tablet, said tablet preferably ensuring immediate release of all three APIs upon administration to a hypertensive patient. If a coated tablet is used, the coating is preferably a film coating that does not affect immediate release of the APIs and has a thickness of about 10 µm to 100 µm, in particular 20 to 70 µm.

That is, in a preferred embodiment, the fixed combination medicinal product to be used in the invention is formulated as an immediate release monolayer tablet. In such a case, the immediate release monolayer tablet preferably comprises an intragranular phase and an extragranular phase, wherein the intragranular phase contains amorphous telmisartan and the extragranular phase contains both amlodipine and indapamide.

An immediate release monolayer tablet meeting such structural requirements is preferably manufactured by a method comprising the steps of:
(i) granulating telmisartan in the presence of at least one filler and optionally other pharmaceutically acceptable excipients so as to obtain telmisartan-containing granules;
(ii) mixing the telmisartan-containing granules with amlodipine, indapamide, at least one disintegrant and optionally other pharmaceutically acceptable excipients so as to obtain a blend;
(iii) processing the blend so as to obtain an immediate release monolayer tablet; and
(iv) optionally coating the immediate release monolayer tablet.

Step (i) preferably comprises wet granulating, in particular fluid bed granulating, telmisartan with a granulation liquid comprising an alkaline agent. As the alkaline agent, an alkaline metal hydroxide such as sodium hydroxide or a basic amino acid such as arginine can be employed.

Step (ii) can be carried out in accordance with conventional techniques, e.g. by dry-mixing the individual components using a mixing device, such as a free-fall-mixer, so as to provide a blend which is essentially homogeneous. Blending can be carried out e.g. for 1 to 30 minutes, in particular 2 to 10 minutes. If necessary, the blend may further be sieved before proceeding to step (iii), e.g. with a sieve having a mesh size of 25 to 1000 µm, in particular 100 to 600 µm.

Step (iii) preferably comprises compressing the blend under a load of 5 to 20 kN, in particular 8 to 14 kN, using a tablet press. Suitable tablet presses are commercially available, for instance, from Fette Compacting GmbH (Schwarzenbek, Germany), Korsch AG (Berlin, Germany) and Riva S.A. (Buenos Aires, Argentina).

Step (iv) is optional and can be carried out, for example, to protect the APIs from early dissolution or undesired decomposition. Excipients for forming a coating are known to a person skilled in the art and comprise, for instance, binders, disintegrants and fillers as disclosed in US 2006/0045865 A1.

### EXAMPLES

The invention will now be explained in more detail based on the following examples which are not intended to limit the scope of the claims.

### Example 1

Telmisartan-containing granules were prepared by dissolving free telmisartan together with Povidone K30 in an aqueous granulation liquid having a pH of about 12, and spraying the granulation liquid obtained thereby onto a mixture of mannitol, meglumine and microcrystalline cellulose using a fluid bed granulator. The dried telmisartan-containing granules were mixed with amlodipine besylate, free indapamide, croscarmellose sodium, magnesium stearate and microcrystalline cellulose so as to obtain a blend, and the blend was compressed using a tablet press.

By using the aforementioned process, immediate release monolayer tablets comprising amorphous telmisartan as an intragranular phase and comprising both crystalline amlodipine and crystalline indapamide as an extragranular phase were obtained. By suitably adjusting the amounts of free telmisartan, amlodipine besylate and free indapamide, immediate release monolayer tablets in accordance with embodiments (a) to (c) described above were obtained. Table 1 shows a summary of the three different immediate release monolayer tablets.

**Table 1**

| Amount of API (in terms of free API) | Monolayer tablet of embodiment (a) | Monolayer tablet of embodiment (b) | Monolayer tablet of embodiment (c) |
|---|---|---|---|
| Telmisartan [mg] | 40 | 80 | 80 |
| Amlodipine [mg] | 5 | 5 | 10 |
| Indapamide [mg] | 2.5 | 2.5 | 2.5 |

### Example 2

A 16-week multicenter, randomized, double-blind phase III study with 505 subjects across 10 treatment groups to evaluate the efficacy and safety effects of triple combinations of telmisartan, amlodipine and indapamide (dosage strengths: T40/A5/I2.5 mg, T80/A5/I2.5 mg and T80/A10/I2.5 mg) compared to dual combinations of telmisartan, amlodipine and indapamide is carried out. The subjects are newly diagnosed patients with essential hypertension or are non-responders to their current antihypertensive therapy.

The triple combinations of telmisartan, amlodipine and indapamide are provided as single-pill combination medicinal products (namely immediate release monolayer tablets obtained according to Example 1), whereas the dual combinations of telmisartan, amlodipine and indapamide are provided as multi-pill formulations In any of the combinations, the active pharmaceutical ingredients are provided as immediate release (IR).

### Study design

The study adapts a single active treatment period factorial design to include non-responders and newly diagnosed patients with essential hypertension. Eligible participants are males or non-child-bearing females with essential hypertension whose sitting diastolic blood pressure (SiDBP) is between 95 and 109 mmHg and sitting systolic blood pressure (SiSBP) is between 145 and 179 mmHg.

### Screening visit (Visit 1)

All participants provide written informed consent. During this visit, the following assessments are performed:
- physical examination including vitals and body measurements
- previous medical history
- prior and concomitant medications are recorded
- females of child-bearing potential have to undergo a pregnancy test
- laboratory test (haematology, glycated haemoglobin, liver enzymes, serum lipids, biochemistry and urinalysis)
- 12-lead electrocardiogram (ECG)

The participants are also introduced to the diet recommended by the clinical guidelines of the European Society of Cardiology (ESC) in the run-in period. The recommended lifestyle measures that have been shown to reduce BP are salt restriction, moderation of alcohol consumption, high consumption of vegetables and fruits, weight reduction, smoking cessation and maintaining an ideal body weight, and regular physical activity.

The study is conducted in accordance with the current Good Clinical Practice.

### Placebo run-in period (2 weeks +/-2 days)

All eligible participants are asked to discontinue their current BP-lowering drug(s) and undergo run-in period for 2 weeks. They are assigned to placebo treatment. Participants are advised to measure and monitor their BP at home with a standardized BP measuring device on a daily basis. Participants are instructed to call to the investigator remotely after one week of run-in period. During this remote communication, BP measurements are reported. Also any possible newly introduced concomitant therapy is reported. If during the run-in period, participants have uncontrolled BP (i.e., sitting systolic >179 mm Hg and sitting diastolic > 109 mm Hg), rescue medication (Perindopril 4 mg QD) is prescribed. If a participant takes the rescue treatment during the run-in period, the participant is considered as a screening failure and is not randomized.

Any serious treatment emergent adverse events (serious TEAEs) are reported and recorded. TEAEs are defined as any untoward event not present before the administration of the study drug or already present but worsened in either intensity or frequency during treatment.

### Double-blind treatment period (16 weeks +/- 2 days)

Participants still eligible after the run-in period, i.e. those who are non-responder participants (whose sitting diastolic blood pressure (SiDBP) is between 95 and 109 mmHg and sitting systolic blood pressure (SiSBP) is between 145 and 179 mmHg with a difference in SiSBP <20 mmHg and of SiDBP of <10 mmHg before and after the run-in period and who consented to participate) are allocated in a double-blind fashion to one of the 10 randomized treatment groups.

The sponsor pre-packages the study drug combination and all of the tablets for corresponding placebo, double combination and triple combination therapies used in study in a blinded fashion. For this purpose, DBcaps^{®} are used to ensure that the investigator, study personnel and participants are kept double blinded to the treatment assignment throughout the study, except for run-in period which is single blinded. DBcaps^{®} capsules are developed with a tamper-evident design to specifically address the clinical trial challenges of testing without bias.

Based on the randomization group, participants are instructed to take the dispensed drug once daily at the same time after a meal in the morning for 16 weeks. If, during the active treatment period, participants have uncontrolled BP (i.e., sitting systolic >179 mm Hg and sitting diastolic > 109 mm Hg), rescue medication (perindopril 4 mg QD) is allowed. The participant is recommended to measure the BP daily and if the BP is lower than 150/110 mm Hg for 3 consecutive days, the participant should stop taking rescue medication.

During this active period, participants are scheduled to come to the clinic for visits after every 8 weeks starting from week 0 (baseline), 8 and 16. The following assessments are performed:
- BP measurements (weeks 0 (baseline), weeks 8 and 16)
- Laboratory test (haematology, biochemistry, liver enzymes, serum lipids and urinalysis - weeks 0 (baseline), 8 and 16)
- 12 lead ECG (week 8 and week 16)
- Serious TEAEs

The total number of in clinic visits from screening to end of treatment is 4 visits:
1. Screening visit - Week -2
2. Randomisation visit - Week 0-1
3. Week 8-9 visit
4. Week 16-17 visit (EOT)

In between the in-clinic visits, participants are scheduled for virtual visits (telephone calls) at weeks 4 and 12 to assess participant safety, concomitant medications and any TEAE/serious TEAEs. Reference baseline is defined as the status of participants after the 2-week run-in period, where participants' eligibility to enter the double-blind treatment period was examined. Compliance is assessed by investigators based on the rate of return and amount of administration of investigational products.

### Blinding and randomization

Randomization is generated using an IRTS (Interactive Response Technology System). Participants are equally randomized to one of ten (10) treatment groups to receive various combination doses of telmisartan, amlodipine and indapamide. Eligible participants are randomized at Randomisation visit on Day 0. Table 2 shows the ten treatment groups.

**Table 2**

| Treatment Group | Combination Doses [mg] |
|---|---|
| 1 | T40, A5 |
| 2 | T80, A5 |
| 3 | T80, A10 |
| 4 | A5, I2.5 |
| 5 | A10, I2.5 |
| 6 | T40, I2.5 |
| 7 | T80, I2.5 |
| 8 | T40, A5, I2.5 |
| 9 | T80, A5, I2.5 |
| 10 | T80, A10, I2.5 |

The treatment groups above represent the combination of 3 strengths of drug: 40/80 mg of telmisartan, 5/10 mg of amlodipine, and 2.5 mg of indapamide immediate release. The study randomizes twice as many participants to the triple groups compared to the dual groups in a 1:1:1:1:1:1:1:2:2:2 ratio (as per treatment group order above).

To maintain the double-blind principle further, DB caps^{®} are used so the investigator, study personnel and participants are kept blinded to the treatment assignment throughout the study. DB caps^{®} capsules are developed with a tamper-evident design to specifically address the clinical trial challenges of testing without bias. The wider diameter of opaque DB caps^{®} capsules allows containment and blinding of large-diameter or uniquely shaped tablets or other comparator products, while their shorter length makes them easier to swallow.

### Inclusion criteria

The inclusion criteria are as follows.
1. Provided signed consent to participate in the trial.
2. Adult between 18 and 80 years.
3. Essential hypertension whose sitting diastolic blood pressure (SiDBP) is between 95 and 109 mmHg.
4. Essential hypertension whose sitting systolic blood pressure (SiSBP) is between 145 and 179 mmHg.

### At randomization visit (Day 0):

5. Essential hypertension with SiDBP between 95 and 109 mmHg.
6. Essential hypertension with SiSBP between 145 and 179 mmHg.
7. Difference in SiSBP between measurements at Visit 1 and Visit 2 of 20 mmHg and/or SiDBP 10 mmHg or less.
8. Drug compliance of 80% and more in the run-in period.

### Exclusion criteria

The exclusion criteria are as follows.
1. Participants with SiDBP ≥ 110 mm Hg and SiSBP ≥ 180 mm Hg.
2. Participants taking 4 or more antihypertensive drugs at signing the informed consent form.
3. Pregnant or had a positive pregnancy test or unwilling to undertake a pregnancy test during the trial and up to 30 days after the discontinuation of the trial medication or breastfeeding or of childbearing age and not using an acceptable method of contraception. Acceptable methods of birth control include hormonal prescription oral contraceptives, contraceptive injections, contraceptive patch, intrauterine device, double-barrier method (e.g. condoms, diaphragm, or cervical cap with spermicidal foam, cream, or gel), or male partner sterilization. Contraception should be used for at least 1 month before the screening visit and until the end of trial participation.
4. Contraindication, including hypersensitivity (e.g. anaphylaxis or angioedema), to the active run-in treatment or to any of the trial medication options in the four randomized groups.
5. Current/history of transient ischemic attack, stroke, or hypertensive encephalopathy.
6. Current/history of acute coronary syndrome, unstable angina, myocardial infarction, percutaneous transluminal coronary revascularization, or coronary artery bypass graft.
7. Current atrial fibrillation. Participants with a history of paroxysmal atrial fibrillation are potentially eligible as long as there has been no episode in the last 3 months, while participants with a history of persistent or permanent atrial fibrillation are not eligible.
8. Current/history of congestive heart failure.
9. Current/history of a known secondary cause of hypertension, such as primary aldosteronism, renal artery stenosis, pheochromocytoma, or Cushing's syndrome.
10. Current/history of substantially uncontrolled diabetes mellitus (HbA1c > 11.0%) within last three months.
11. Current/history of end-stage renal disease, anuria, or estimated glomerular filtration rate (eGFR) ≤ 60 ml/min/1.73m².
12. Serum creatinine level ≥ or = 1.5 times ULN.
13. Electrolyte levels that would be regarded as contraindications for any of the potential treatment groups e.g. serum sodium ≤ 132 mmol/l or ≥ 148 mmol/l serum potassium ≤3.1 mmol/l or ≥ 5.6 mmol/l.
14. Current/history of aspartate aminotransferase (AST) or alanine aminotransferase (ALT) ≥ 3 times the upper limit of normal range within 6 months.
15. Current concomitant illness or physical impairment or mental condition that in the judgment of the investigator could interfere with the effective conduct of the trial or constitutes a significant risk to the participants' well-being.
16. Currently taking or might need during the trial, a concomitant treatment that is known to interact with one or more of the trial medications: digoxin, lithium, diabetics receiving aliskiren, moderate and strong CYP3A4 inhibitors (e.g. ritonavir, ketoconazole, diltiazem), simvastatin > 20 mg/day, immunosuppressants.
17. Might need treatment with drugs that are prohibited during the trial: other antihypertensive drugs, endothelin receptor antagonists, neprilysin inhibitors, or other drugs that may affect BP.
18. Current surgical or medical condition that might significantly alter the absorption, distribution, metabolism, or excretion of trial drugs such as prior major gastrointestinal tract surgery (e.g. gastrectomy, lap band, or bowel resection) or acute flare of inflammatory bowel disease within one year.
19. Participated in any investigative drug or device trial within the previous 30 days.
20. History of alcohol or drug abuse within 12 months.

### At randomization visit:

1. Unable to adhere to the trial procedures during the run-in period.
2. Any condition which in the investigator's judgment may compromise the safety of the participant.
3. Any abnormal laboratory value which in the judgment of the investigator could interfere with the effective conduct of the trial or constitutes a significant risk to the participant's well-being.
4. Fulfilling any of the exclusion criteria mentioned for the screening visit, when verified again at randomization visit.

### Study endpoints

### Primary endpoint

The primary endpoint is the reduction from the baseline in mean SiSBP (Week 16).

### Secondary endpoints

The secondary endpoints are the following:
1. Reduction from the baseline in mean SiDBP (Week 16)
2. Reduction from the baseline in SiDBP (Week 8)
3. Reduction from the baseline in mean SiSBP (Week 8)
4. SiDBP control rate at Week 8 and 16
   SiDBP control rate: the proportion of participants with controlled SiDBP of less than 90 mmHg after Week 8 and 16
5. SiSBP control rate at Week 8 and 16
   SiSBP control rate: the proportion of participants with controlled SiSBP of less than 140 mmHg after Week 8 and 16
6. SiDBP response rate at Week 8 and 16
   SiDBP response rate: the proportion of participants who achieved an adequate response in SiDBP (< 90 mmHg or/and reduction from reference baseline ≥ 10 mmHg) after Week 8 and 16
7. SiSBP response rate at Week 8 and 16
   SiSBP response rate: the proportion of participants who achieved an adequate response in SiSBP (< 140 mmHg or/and reduction from reference baseline ≥ 20 mmHg) after Week 8 and 16
8. Sitting Blood Pressure (SiBP) normalization rate at Week 8 and 16
   Sitting blood pressure (SiBP) normalization: The proportion of participants whose blood pressure was within normalization criterion (SiSBP/SiDBP <140/90mm Hg) after Week 8 and 16

### Safety endpoints

The safety endpoints are the following:
1. Overall incidence of Treatment Emergent Adverse Events (TEAEs)
2. Incidence of TEAEs stratified by specific type of TEAE
3. Total (and percentage) number of participants with Serious TEAEs from Week 0 to Week 16
2. Total number (and percentage) of deaths from Week 0 to Week 16.
3. Percentage of participants with orthostatic hypotension from baseline to Week 16 (any time between the day 0 and week 16). Orthostatic hypotension is defined as a decrease in systolic blood pressure of 20 mmHg or a decrease in diastolic blood pressure of 10 mmHg within three minutes of standing when compared with blood pressure from the sitting or supine position.
4. Percentage of participants with serum sodium concentration above 145 mmol/l at Week 16.
5. Percentage of participants with serum potassium concentration below 3.5 mmol/l at Week 16.
6. Percentage of participants with eGFR drop of over 10% from baseline to Week 16.

## Claims

1. A fixed combination medicinal product for use in treating essential hypertension or/and reducing the risk of development of a condition associated with essential hypertension, wherein:
(i) the fixed combination medicinal product comprises:
(a) telmisartan at a dose of 40 mg, amlodipine at a dose of 5 mg, and indapamide at a dose of 1.5 mg for modified release or at a dose of 2.5 mg for immediate release;
or
(b) telmisartan at a dose of 80 mg, amlodipine at a dose of 5 mg, and indapamide at a dose of 1.5 mg for modified release or at a dose of 2.5 mg for immediate release; or
(c) telmisartan at a dose of 80 mg, amlodipine at a dose of 10 mg, and indapamide at a dose of 1.5 mg for modified release or at a dose of 2.5 mg for immediate release;
(ii) the fixed combination medicinal product is administered once or twice a day; and
(iii) the fixed combination medicinal product is used as:
(α) a first-line antihypertensive therapy for a hypertensive patient; or
(β) a second-line antihypertensive therapy for a hypertensive patient who is a non-responder to a previous antihypertensive therapy; or
(γ) a substitution antihypertensive therapy for a hypertensive patient whose blood pressure is adequately controlled by a previous antihypertensive therapy.

2. The fixed combination medicinal product for use according to claim 1, wherein the fixed combination medicinal product is used as a substitution antihypertensive therapy for a hypertensive patient whose blood pressure is adequately controlled by a previous antihypertensive therapy, in particular a previous second-line antihypertensive therapy.

3. The fixed combination medicinal product for use according to claim 1 or 2, wherein the previous antihypertensive therapy comprises administration of at least one antihypertensive agent selected from telmisartan, amlodipine and indapamide.

4. The fixed combination medicinal product or use according to any one of claims 1 to 3, wherein the previous antihypertensive therapy comprises administration of a fixed or non-fixed combination of telmisartan and amlodipine, a fixed or non-fixed combination of telmisartan and indapamide, or a fixed or non-fixed combination of amlodipine and indapamide.

5. The fixed combination medicinal product or use according to any one of claims 1 to 3, wherein the previous antihypertensive therapy comprises administration of a fixed or non-fixed combination of telmisartan, amlodipine and indapamide, in particular a non-fixed combination of telmisartan, amlodipine and indapamide.

6. The fixed combination medicinal product for use according to any one of claims 1 to 5, wherein the hypertensive patient is an adult patient having, without pharmacological interventions, a sitting systolic blood pressure from 140 to 180 mmHg or/and a sitting diastolic blood pressure from 90 to 110 mmHg, in particular a sitting systolic blood pressure from 160 to 179 mmHg or/and a sitting diastolic blood pressure from 100 to 109 mmHg.

7. The fixed combination medicinal product for use according to any one of claims 1 to 6, wherein the hypertensive patient is treated despite having a history of at least one of the following conditions:
• hypertensive encephalopathy, stroke or transient ischemic attack;
• acute coronary syndrome, coronary artery disease, coronary artery bypass graft, myocardial infarction, percutaneous transluminal coronary revascularization or unstable angina;
• persistent or permanent atrial fibrillation;
• congestive heart failure;
• primary aldosteronism, renal artery stenosis, pheochromocytoma or Cushing's syndrome;
• anuria, end-stage renal disease, or estimated glomerular filtration rate (eGFR) of ≤ 60 ml/min/1.73m²;
• serum creatinine level of ≥ 1.5 times the upper limit of normal range;
• aspartate aminotransferase level or alanine aminotransferase level ≥ 3 times the upper limit of normal range; and
• alcohol or drug abuse.

8. The fixed combination medicinal product for use according to any one of claims 1 to 7, wherein the hypertensive patient is a prediabetic patient or a diabetic patient, in particular a type 2 diabetic patient.

9. The fixed combination medicinal product for use according to claim 8, wherein the diabetic patient has a history of substantially uncontrolled diabetes, in particular a HbA1c level of > 11.0%, within the last three months before the start of treatment with the fixed combination medicinal product.

10. The fixed combination medicinal product for use according to any one of claims 1 to 9, wherein administration of the fixed combination medicinal product is interrupted when the hypertensive patient experiences at least one of the following conditions:
• pregnancy or a positive pregnancy test;
• serum sodium level of ≤ 132 mmol/l or ≥ 148 mmol/l;
• serum potassium level of ≤ 3.1 mmol/l or ≥ 5.6 mmol/l; and
• emergency treatment with digoxin, lithium, aliskiren, ritonavir, ketoconazole, diltiazem, simvastatin or an immunosuppressant.

11. The fixed combination medicinal product for use according to any one of claims 1 to 10, wherein the fixed combination medicinal product comprises telmisartan, amlodipine and indapamide as the sole active ingredients.

12. The fixed combination medicinal product for use according to any one of claims 1 to 11, wherein the fixed combination medicinal product is formulated as a tablet, in particular as an immediate release monolayer tablet.

13. The fixed combination medicinal product for use according to claim 12, wherein the tablet is obtained by a wet-granulation process and comprises amorphous telmisartan as an intragranular phase and comprises both amlodipine and indapamide as an extragranular phase.

14. The fixed combination medicinal product for use according to any one of claims 1 to 13, wherein the condition associated with essential hypertension is a cardiovascular event, in particular a cardiovascular event selected from acute coronary syndrome, coronary artery disease, coronary artery bypass graft, hypertensive encephalopathy, myocardial infarction, percutaneous transluminal coronary revascularization, stroke, transient ischemic attack and unstable angina.

15. The fixed combination medicinal product for use according to any one of claims 1 to 14, wherein the fixed combination medicinal product is administered once a day.
